(19) Europäisches Patentamt
European Patent Office
Office européen des brevets

(11) Publication number: **0 307 179 A2**

(12) # EUROPEAN PATENT APPLICATION

(21) Application number: 88308264.6

(22) Date of filing: 07.09.88

(51) Int. Cl.4: C07B 59/00 , C07D 487/14 , C07D 491/22 , A61K 43/00 , //(C07D487/14,209:00,209:00, 203:00)

(30) Priority: 10.09.87 JP 227094/87

(43) Date of publication of application: 15.03.89 Bulletin 89/11

(84) Designated Contracting States: DE FR GB

(71) Applicant: KYOWA HAKKO KOGYO KABUSHIKI KAISHA
6-1, Ohte-machi 1-chome
Chiyoda-ku Tokyo 100(JP)

(72) Inventor: Masaji, Kasai
3-12-15, Matsugaoka Kugenuma
Fujisawa-shi Kanagawa-ken(JP)
Inventor: Yutaka Kanda
Daini Kenyuryo 4-17-9, Morino
Machida-shi Tokyo-to(JP)
Inventor: Makoto Morimoto
203-5, Shimo-togari Nagaizumi-cho
Sunto-gun Shizuoka-ken(JP)
Inventor: Shiro, Akinaga
1188, Shimo-togari Nagaizumi-cho
Sunto-gun Shizuoka-ken(JP)
Inventor: Yotaka, Saito
12-203, Popuragaoka Koopu 2-18-1, Naruse
Machida-shi Tokyo-to(JP)
Inventor: Motomichi, Kono
410-1, Nameri Nagaizumi-cho
Sunto-gun Shizuoka-ken(JP)

(74) Representative: Hildyard, Edward Martin et al
Frank B. Dehn & Co. European Patent Attorneys Imperial House 15-19 Kingsway
London WC2B 6UZ(GB)

(54) **Mitomycins and processes for their preparation.**

(57) Mitomycins which are isotopically labelled with $^2H$ or $^3H$ in the $C_6$-methyl group are of interest for metabolic studies. The compounds may be prepared from novel 6-methylenemitomycins (the latter derived from novel 6-phenylselenomitomycins) by reduction with labelled borohydride.

EP 0 307 179 A2

## Mitomycins and processes for their preparation

The present invention relates to mitomycins labelled with isotopes, intermediates useful for their synthesis, and processes for their preparation.

When pharmaceutical agents are used for research, development or clinical purposes, it is important to know their metabolic fate in the living body. For this purpose, it was previously known to use agents labelled with isotopes. In the field of labelled mitomycins, for example, the following mitomycins are known:-

1) Mitomycin C labelled with tritium ($^3H$) by the Wilzbach's method [Biochemistry, 4, 196-200 (1965)]. However, the location of the isotope is not specified.

2) Porfiromycin viz. 1a-N-methylmitomycin C, of which 1a-N-methyl group is labelled with $^{14}C$ or $^3H$ [for example, Biochemistry, 13, 4878-4887 (1974); J. Labelled Compd. Radiopharm., 21, 789-791 (1984)].

A process for the preparation of mitomycin C labelled with isotopes at specified positions is known, in which, by means of biosynthesis, amino acids labelled with isotopes at specified positions such as, for example, [methyl - $^{14}C$]-methionine and [guanido-$^{14}C$]-L-arginine are used as the precursors of mitomycin C and are incorporated into mitomycin C to label its 9a-O-methyl group and carbamoyl groups respectively with said isotopes [J. Chem. Soc. Chem. Commun., 301-302 (1971)].

However, it has been reported that known labelled mitomycins are still insufficient for tracing the behaviour of mitomycin in the living body because the location of the labelling is not optimally selected or because the location is not stable during metabolism of the compound [cf "Mitomycins and the progress of tumor-chemotherapy", page 98 (1984), edited by Tetsuo Taguchi and published by Kyowa Kikaku Tsushin in Japanese version].

Thus, for the purpose of tracing the behaviour of mitomycins in the living body exactly, it has been desired to provide improved mitomycins labelled with a desired isotope at an optimal position which is stable during the metabolism of mitomycins.

The present invention is based upon the discovery that labelled mitomycins having excellent properties may be obtained by substituting a hydrogen atom of the methyl group located at the $C_6$-position of mitomycins.

According to one feature of the present invention, there are provided mitomycins, of which methyl group located at the 6-position is labelled with an isotope, and intermediates therefore, represented by the following formula (I) :-

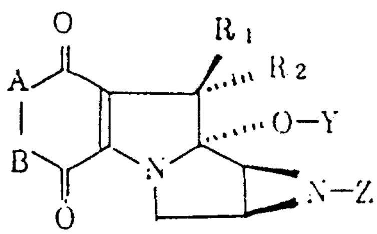

(I)

(wherein
-A-B- represents one member selected from

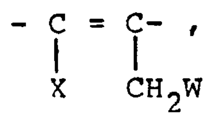

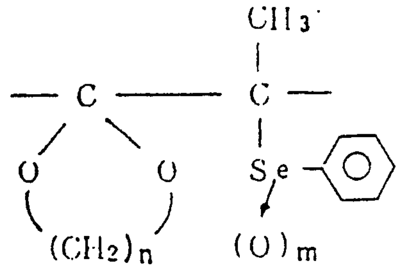
,
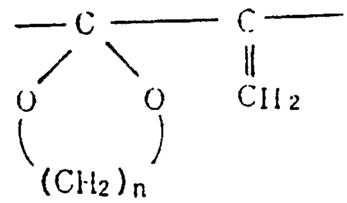

and

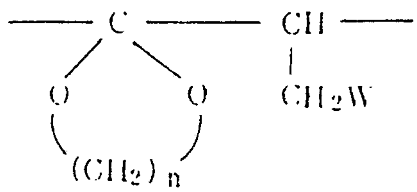

[wherein W represents deuterium ($^2H$) or tritium ($^3H$);
X represents a methoxy or amino group;
n is an integer of 2 or 3;
m is an interger of 0 or 1;
one of $R_1$ and $R_2$ represents a carbamoyloxymethyl group and the other represents a hydrogen atom;
optionally $R_1$ and $R_2$ are combined together and represent a methylene group ($=CH_2$);
Y represents a hydrogen atom or a methyl group;
and Z represents a hydrogen atom, a methyl group or an acyl group).

The compounds of the present invention are referred to as Compounds (I) hereinafter. Compounds represented by other formulae are also designated similarly. The isotopically labelled compound of the invention may be formulated into pharmaceutical compositions by admixture with physiologically acceptable carriers in a manner known perse. Such compositions may be used to trace the metabolism fate of the mitomycin in a mammal or mammalian cell.

The present invention also provides processes for the preparation of Compounds (I), intermediates for the synthesis of the same and processes for the preparation of such intermediates.

With regard to the definition of Z in the formula (I), examples of the acyl group (R-CO) include those wherein R is lower alkyl having 1-4 carbon atoms (such as, for example, methyl, ethyl, propyl and butyl groups) and those wherein R is an unsubstituted or substituted phenyl group (such as, for example, phenyl, tolyl, xylyl, 4-chlorophenyl and 4-methoxyphenyl groups).

Compounds (I) may be prepared by the following processes:-

[Process- A]

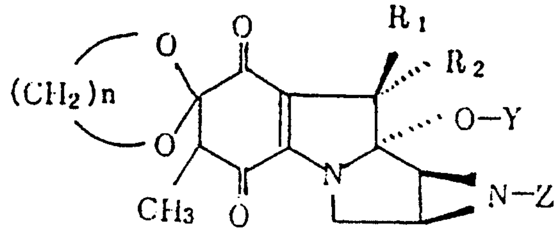

(II)

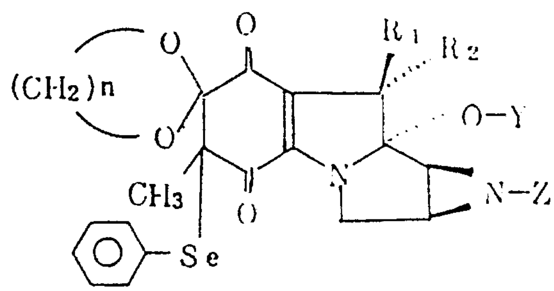

(I-2a)

(wherein $R_1$, $R_2$, Y, Z and n are as hereinbefore defined)

Compounds (I-2a) may be prepared by the reaction of Compound (II) [disclosed in Japanese Patent Application No. 71115/87] with Compound (III) viz. a selenenylating agent:-
(wherein $R_3$ is selected from halogen atoms such as chlorine, bromine and iodine; and organic imide residues such as succinimide and phthalimide) in an inert solvent in the presence of a base.

The solvents which may be used for the reaction are exemplified by ether, tetrahydrofuran, methylene chloride and chloroform, which may be used alone or in admixture.

Suitable bases which may be used for the reaction are exemplified by triethylamine, pyridine and other organic bases. It is preferred to use more than an equimolar amount of the base, for example 4-10 moles per mole of Compound (III).

Usually, the reaction may be effected at a temperature within a range of from 0 to 30°C for a period of from 20 minutes to 16 hours.

After completion of the reaction, the reaction solution may be concentrated without work-up, although it is possible, if desired, to extract the reaction solution with a non-aqueous solvent such as, for example, methylene chloride, chloroform or ethyl acetate. The extracted solution may be washed in conventional manner, followed by further concentration. The concentrated solution may be purified, for example, by column chromatography, preparative thin layer chromatography or recrystallization.

[Process- B] Compound (I-2a)

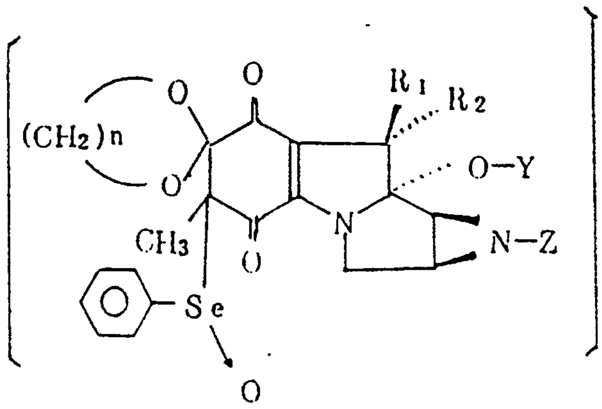

(I-2b)

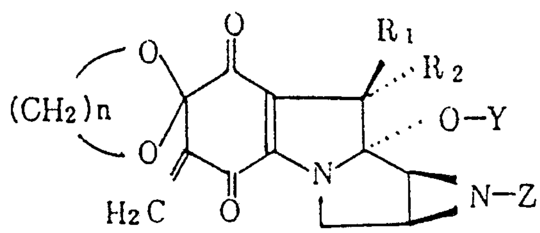

(I-3)

(wherein $R_1$, $R_2$, Y, Z and n are as hereinbefore defined).

Compounds (I-3) may be obtained from Compound (I-2a) through Compound (I-2b) viz. an intermediate by the reaction of Compound (I-2a) with an oxidizing agent in an inert solvent under neutral or basic conditions. The solvents which may be used for the reaction are exemplified by ether, tetrahydrofuran, methylene chloride and chloroform, although it is possible, if desired, to use alcohol-type solvents such as, for example, methanol and ethanol.

The bases which may be used to carry out the reaction under basic conditions include, for example, organic bases such as triethylamine and pyridine. It is preferred to use a base in a slight excess, for example, 2 to 40 moles per mole of the oxidizing agent used.

Examples of suitable oxidizing agents include perorganic acids such as metachloroperbenzoic acid and peracetic acid, although it is possible to use sodium metaperiodate, hydrogen peroxide and ozone. The oxidizing agent may be used, for example, in an amount of 1-2 moles per mole of Compound (I-2a).

The reaction may be completed, for example, at a temperature of from 0 to 30 °C within a period of from 10 minutes to one hour.

Usually, the resultant compound (I-3) may be used for the subsequent reaction without further work-up.

[Process-C] Compound (I-3)

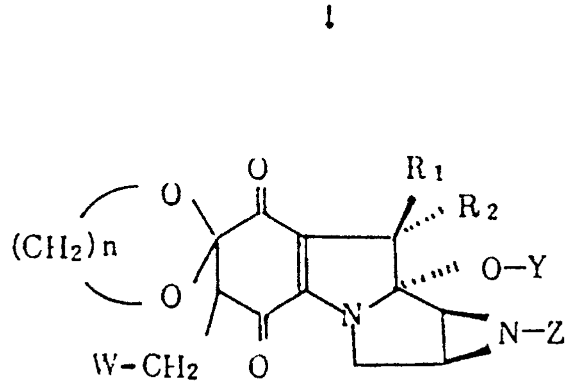

(I-4)

(wherein $R_1$, $R_2$, W, Y, Z and n are as hereinbefore defined).

Compounds (I-4) may be prepared by the reaction of Compound (I-3) with a complex hydride, especially an alkali metal borohydride such as sodium borohydride, labelled with an isotope such as deuterium ($^2H$) or tritium ($^3H$), in a solvent. The reaction may be effected e.g. by adding sodium borohydride labelled with an isotope to the reaction solution in a solvent which may be the same as the solvent used for the preparation of Compound (I-3) in the above-mentioned Process-B, although it is possible, if desired, to use Compound (I-3) isolated from such a solvent.

The amount of sodium borohydride may be, for example, 1-4 moles per mole of Compound (I-2a). The reaction may be completed at a temperature of from 0 to 30°C within a period of from 10 minutes to 3 hours.

After completion of the reaction, the reaction solution may be neutralized, although it is possible, if desired, to add a buffer solution to the reaction solution. Then, the solution may be extracted with a non-aqueous solvent such as, for example, methylene chloride, chloroform or ethyl acetate. The extracted solution may be washed, for example, with water, aqueous sodium bicarbonate or saturated brine and concentrated. Then, the desired compound may be purified, for example, by column chromatography, preparative thin layer chromatography or recrystallization.

[Process-D] Compound (I-4)

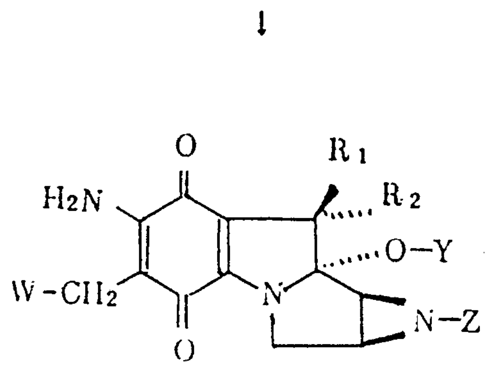

(I-1a)

(wherein $R_1$, $R_2$, W, Y and Z are as hereinbefore defined).

Compounds (I-1a) may be prepared by the reaction of Compound (I-4) with ammonia in an inert solvent.

Any and all solvents capable of dissolving Compound (I-4) may be used for the reaction, which are exemplified by alcohol-type solvents such as methanol and ethanol; ether-type solvents such as ether and tetrahydrofuran; haloalkanes such as methylene chloride and chloroform; acetonirile, dimethylformamide and dimethylsulfoxide. These solvents may be used alone or in admixture.

Usually, the reaction may be completed at a temperature of from 0 to 30 °C for one hour to 20 hours.

After completion of the reaction, the reaction solution may be concentrated without work-up, although it is possible, if desired, to extract the reaction solution with a non-aqueous solvent such as, for example, chloroform or ethyl acetate, followed by concentration. Then, the desired compound may be purified, for example, by column chromatography, preparative thin layer chromatography, or recrystallization.

[Process-E] Compound (I-4)

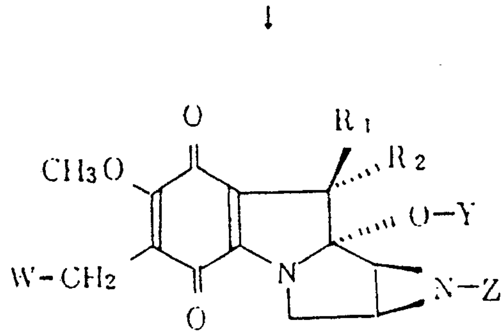

(I-1b)

(wherein $R_1$, $R_2$, W, Y and Z are as hereinbefore defined)

Compounds (I-1b) may be prepared by the reaction of Compound (1-4) in methanol in the presence of a base. The bases which may be used for the reaction are represented by the formula:-

ROM

(wherein R represents a lower alkyl group having 1-4 carbon atoms: and M represents an alkali metal or an alkaline earth metal).

Examples of suitable bases include hydroxides, carbonates and bicarbonates of alkali metals and alkaline earth metals, as well as tertiary amines and quarternary ammonium hydroxides.

The base may be used in an amount of e.g. 0.001 to 10 moles, preferably 0.01 to 3 moles per mole of Compound (I-4). The reaction may be completed at a temperature of from 20 to 30 °C for a period of 2 to 24 hours. The completion of the reaction may easily be detected by thin layer chromatography.

After completion of the reaction, the reaction solution may be neutralized, for example, by using organic acids such as acetic acid or propionic acid; inorganic acids such as hydrochloric acid or sulfuric acid; or by dry ice. Then, extraction may be effected by non-aqueous solvents such as methylene chloride, chloroform or ethyl acetate. For example, after washing with water or saturated solution of sodium chloride the extracted solution may be concentrated and purified, for example, by column chromatography, preparative thin layer chromatography or recrystallization.

In the case where Compound (I-4), wherein Z represents an acyl group, is prepared by Processes D and E, a deacylation reaction may occur simultaneously to result in the corresponding Compounds (I-1a) and (I-1b), wherein Z is hydrogen, respectively.

The following Examples illustrate the present invention. The physico-chemical characteristics of the products were determined by means of the following instruments:-

MS : Hitachi M-80B (commercial product of Hitachi Seisakusho, Japan) by the EI and SI methods.

$^1$H-NMR: Bruker AM400 (400 MHz; measured in deuterochloroform).

IR : IR-810 (commerical product of Nihon Bunko K.K., Japan) by KBr method.

TLC : Silica gel Art 5719 (commercial product of Merck AG. West Germany).

Example 1

1a-N-acetyl-7-demethoxy-6,7-dihydro-7-ethylenedioxy-6-phenylselenomitomycin A [Compound I; compound of the formula (I-2a) wherein $R_1$ = $CH_2OCONH_2$, $R_2$ = H, Y = $CH_3$, Z = $COCH_3$ and n = 2]:

120 mg of 1a-N-acetyl-7-demethoxy-6,7-dihydro-7-ethylenedioxymitomycin A [Compound (II-I); compound of the formula (II) wherein $R_1$ = $CH_2OCONH_2$, $R_2$ = H, Y = $CH_3$, Z = $COCH_3$ and n = 2] was dissolved in tetrahydrofuran (4.0 ml). After adding triethylamine (0.3 ml), the reaction solution was stirred at a temperature of 25°C for 30 minutes. Then phenylselenenyl bromide (67 mg) was added thereto, followed by stirring the mixture at a temperature of 25°C for 30 minutes. Then further phenylselenenyl bromide (60 mg) was added thereto, followed by stirring the mixture for 20 minutes.

Then, the solvent was removed from the mixture by evaporation under reduced pressure. The residue was subjected to silica gel column chromatography using a solvent system of chloroform/methanol (97:3). The yellow fractions were collected and combined. The combined fractions were evaporated under reduced pressure to remove the solvent. The residue was dissolved in a small amount of chloroform. By adding n-hexane, the material was precipitated as a powder. After removal of the solvent by evaporation under reduced pressure, the material was well dried in vacuo at a temperature of 25°C to obtain Compound 1 (149 mg; yield 91%) as a yellow powder. The resultant Compound 1 was a mixture of compounds having different absolute configurations at the $C_6$ position. A mixture of two compounds in a mole ratio of about 2:1 was observed by $^1$H-NMR [Process-A].

TLC : Rf = 0.49 (chloroform:methanol = 9:1)

SI-MS : m/z, 578 ($M^+$ + 2), $C_{25}H_{27}N_3O_8Se$ = 576

IR:$cm^{-1}$ 3460, 2970, 1725, 1688, 1656, 1571, 1435, 1377, 1334, 1247, 1205, 1066, 1031, 987, 857, 742

$^1$H-NMR: δ, ppm

Major: 1.45 (3H, S), 2.18 (3H, S), 3.16 (3H, S), 3.27, (1H, dd, J = 4.7, 2.0), 3.39 (1H, dd, J = 13.0, 2.0), 3.51 (1H, d, J = 4.4), 3.80 (1H, dd, J = 11.3, 4.9), 3.84 (1H, d, J = 13.0), 3.95-4.50 (4H, m), 4.28 (1H, t, J = 11.1), 5.15 (1H, dd, J = 11.0, 4.7), 5.80 (2H, bs), 7.24-7.63 (5H, m).

Minor: (main peaks) 1.42 (3H, s), 2.18 (3H, s), 3.22 (1H, dd, J = 4.4, 2.0), 3.26 (3H, s), 3.38 (1H, dd, J = 13.0, 2.2), 3.47 (1H, d, J = 4.4), 3.75 (1H, dd, J = 11.0, 4.7), 4.14 (1H, t, J = 10.8), 4.54 (1H, d, J = 12.9), 4.85 (1H, dd, J = 11.0, 4.7).

Example 2

[$C_6$-$CH_3$, $^2H_1$]mitomycin C [Compound 2; compound of the formula (I-1a) wherein W = $^2$H, $R_1$ = $CH_2OCONH_2$, $R_2$ = Z = H and Y = $CH_3$].

In a mixture of chloroform (3.5 ml) and pyridine (0.5 ml) was dissolved Compound 1 (201 mg) prepared by the method of Example 1. Metachloroperbenzoic acid having a purity of 70% (129 mg) was added to the reaction solution at a temperature of 0°C. After stirring the mixture at the same temperature for 25 minutes, there was obtained a reaction solution containing Compound 3 viz. a compound of the formula (I-3) wherein $R_1$ = $CH_2OCONH_2$, $R_2$ = H, Y = $CH_3$ Z = $COCH_3$ and n = 2 [Process-B].

The following physico-chemical characterisitics of the product revealed the formation of Compound 3:-

SI-MS: m/z, 420 ($M^+$ + 1), $C_{19}H_{21}N_3O_8$ = 419

$^1$H-NMR: δ, ppm (chloroform-$d_1$/pyridine-$d_5$)

2.08 (3H, s), 3.17 (3H, s), 3.24 (1H, dd, J = 4.4, 2.0), 3.50 (1H, d, J = 4.4), 3.48 (1H, dd, J = 13.3, 2.0), 3.79 (1H, dd, J = 11.1, 4.9), 4.17 (1H, t, 10.8), 4.04-4.29 (4H, m), 4.38, (1H, d, J = 13.3), 5.04 (1H, dd, J = 10.8, 4.7), 5.79 (2H, bs), 6.09 (1H, bs), 6.36 (1H, bs).

To the resultant reaction solution were added 7.3 mg of sodium borohydride labelled with $^2$H and 0.2 ml of methanol-$d_4$. After stirring the mixture at a temperature of 0°C for 40 minutes, 1/15M phosphate-buffered solution (pH 4.0) was added thereto.

The water layer was extracted with chloroform. Then the chloroform layer was washed with a solution of sodium bicarbonate and a saturated solution of sodium chloride, followed by drying with anhydrous sodium sulfate. After removal of the solvent by evaporation under reduced pressure, the residue was subjected to silica gel chromatography using a solvent system of chloroform/methanol (97:3). Yellowish pink fractions which showed Rf = 0.52 by silica gel thin layer chromatography using a solvent system of

chloroform/methanol (9:1) were collected. After removal of the solvent by evaporation under reduced pressure, there was obtained Compound 4 viz. a compound of the formula (I-4) wherein $W = {}^2H$, $R_1 = CH_2OCONH_2$, $R_2 = H$, $Y = CH_3$, $Z = COCH_3$ and $n = 2$ [Process-C].

The production of Compound 4 was confirmed by the observed Rf = 0.52, by TLC using a solvent system of chloroform and methanol (9:1), identical with the corresponding Rf value of Compound (II-1).

This compound was dissolved in methanol (0.2 ml) and 6.8M-ammonia/methanol solution (0.5 ml) was added thereto. The mixture was stirred at a temperature of 25°C for 5 hours. After removal of the solvent by evaporation under reduced pressure, the residue was chromatographed on silica gel using a solvent system of chloroform/methanol (9:1) and fractions showing Rf = 0.30 were collected. By recrystallization, from chloroform, purple crystals of [$C_6$-$CH_3$, ${}^2H_1$]-mitomycin C (Compound 2; 30 mg) were obtained with a yield of 26% calculated on the basis of Compound 2 [Process-D]. ${}^1H$-NMR data suggested a mole ratio of deuterated product of about 60%.

EI-MS : m/z, 335 ($M^+$), $C_{15}H_{17}{}^2HN_4O_5 = 335$.

IR : $cm^{-1}$ 3450, 3320, 2950, 1709, 1592, 1543, 1442, 1339, 1219, 1061, 960, 921, 758, 700

${}^1H$-NMR: δ, ppm

about 0.9 (1H, bs), 1.74 (about 1.2H, t, J = 2.2), 1.76 (about 1.2H, s), 2.82 (1H, bd, J = 4.4), 2.89 (1H, d, J = 4.4), 3.21 (3H, s), 3.51 (1H, dd, J = 12.8, 1.7), 3.63 (1H, dd, J = 10.6, 4.4), 4.25 (1H, d, J = 12.8), 4.53 (1H, bt, J = 10.6), 4.71 (1H, dd, J = 10.6, 4.4), 4.71 (2H, bs), 5.20 (2H, bs).

## Example 3

[($C_6$-$CH_3$, ${}^3H_1$]mitomycin C [Compound 5 viz. compound of the formula (I-1a) wherein $W = {}^3H$, $R_1 = CH_2OCONH_2$, $R_2 = Z = H$ and $Y = CH_3$]:

Compound 1 (45 mg) prepared by the method of Example 1 was dissolved in a mixture of chloroform (20 ml) and pyridine (0.5 ml). Metachloroperbenzoic acid having a purity of 70% (30 mg) was added thereto. The mixture was stirred at a temperature of 20°C for 10 minutes (Process-B).

Sodium borohydride (1.9 mg) labelled with tritium (25m Ci of 500 m Ci/mmol) was dissolved in ethanol (0.8 ml) and added to the reaction solution. The solution was stirred at a temperature of 20°C for 15 minutes and diluted with chloroform (2.0 ml). The chloroform solution was treated in a similar manner to that described in Example 2 to obtain Compound 6 viz. a compound of the formula (I-4) wherein $W = {}^3H$, $R_1 = CH_2OCONH_2$, $R_2 = H$, $Y = CH_3$, $Z = COCH_3$ and $n = 2$ [Process-C].

Compound 6 was dissolved in methanol (2.0 ml). Then 6.8M-ammonia-methanol solution (0.5 ml) was added to the reaction solution, which was then treated in a similar manner to that described in Example 2. The reaction product was precipitated as a powder with chloroform/n-hexane to obtain [$C_6$-$CH_3$, ${}^3H_1$]-mitomycin C viz. Compound % (4 mg) in the form of a grey powder with a yield of 15% calculated on the basis of Compound 1 (Process-D).

A relative radio-activity of 16 m Ci/mmol was measured using a Packard Tri-carb 4530 Liquid Scintillation Counter.

## Example 4

[$C_6$-$CH_3$, ${}^2H_1$]mitomycin A [Compound 7 viz. compound of the formula (I-1b) wherein $W = {}^2H$, $R_1 = CH_2OCONH_2$, $R_2 = Z = H$ and $Y = CH_3$]:

In a similar manner to Processes B and C described in Example 2, Compound 4 was prepared. 70 mg of Compound 4 was dissolved in methanol (3.0 ml). Then potassium hydroxide (1.0 mg) was added thereto. After stirring at a temperature of 20°C for 12 hours, the reaction solution was neutralized by addition of small pieces of dry ice. After addition of a saturated solution of sodium chloride, the reaction solution was extracted with chloroform. Anhydrous sodium sulfate was used to dry the chloroform layer. After removal of the solvent by evaporation under reduced pressure, the residue was subjected to silica gel column chromatography using a solvent system of chloroform/methanol (9:5). Reddish purple fractions which showed Rf = 0.49 on silica gel TLC using a solvent system of chloroform/methanol (9:1) were collected. After removal of the solvent by evaporation under reduced pressure, the material was precipitated as a

powder with chloroform/n-hexane. The solvent was removed from the material by evaporation under reduced pressure. After drying in vacuo, there was obtained [$C_6$-$CH_3$, $^2H_1$] mitomycin A viz. Compound 7 (37 mg) in the form of a reddisch purple powder with a yield of 64% [Process-E].

On the basis of $^1$H-NMR data, it was noted that the ratio of $^2$H contained in the product was about 70%.

EI-MS : m/z 350 ($M^+$) $C_{16}H_{18}{}^2HN_3O_6$ = 350

$^1$H-NMR: δ, ppm

about 0.9 (1H, bs), 1.82 (about 1.4H, t, J = 2.2), 1,85 (about 0.9H, s), 2.83 (1H, bs), 2.92 (1H, d, J = 4.2), 3.22 (3H, s), 3.48 (1H, dd, J = 12.8, 1.7), 3.62 (1H, dd, J = 10.6, 4.4), 4.04 (3H, s), 4.05 (1H, d, J = 12.8), 4.56 (1H, t, J = 10.6), 4.74 (1H, dd, J = 10.6, 4.4), 4.89 (2H, bs)

Example 5

[$C_6$-$CH_3$, $^2H_1$]porfiromycin [Compound 8 viz. compound of the formula (I-1a) wherein W = $^2$H, $R_1$ = $CH_2OCONH_2$, $R_2$ = H and Y = Z = $CH_3$]:

850 mg of 7-demethoxy-6,7-dihydro-7-ethylenedioxymitomycin F (Compound II-2) [compound of the formula (II) wherein $R_1$ = $CH_2OCONH_2$, $R_2$ = H, Y = Z = $CH_3$ and n = 2] was dissolved in tetrahydrofuran (20 ml). Triethylamine (1.5 ml) was added thereto. After addition of phenylselenenyl bromide (780 mg), the material was treated in a similar manner to that described in Example 1 to prepare Compound 9 viz. a compound of the formula (I-2a) wherein $R_1$ = $CH_2OCONH_2$, $R_2$ = H, Y = Z = $CH_3$ and n = 2.

Compound 9 formed a yellow powder and was a mixture of compounds whose absolute configurations differ at the $C_6$ position.

Silica gel TLC using a solvent system of chloroform/methanol (9:1) revealed Rf values of 0.72 and 0.67, from which a mole ratio of two isomers of about 6:5 was calculated [Process-A].

SI-MS : m/z 550 ($M^+$ + 2), $C_{24}H_{27}N_3O_7Se$ = 548

$^1$H-NMR: δ, ppm

Major: 1.39 (3H, s), 2.24 (1H, dd, J = 4.7, 2.2), 2.24 (3H, s), 2.27 (1H, d, J = 4.7), 3.26 (3H, s) 3.32 (1H, dd, J = 12.6, 2.2), 3.59 (1H, dd, J = 10.6, 4.4), 4.32 (1H, d, J = 12.6), 4.38 (1H, t, J = 10.6), 4.00-4.50 (4H, m), 4.57 (1H, dd, J = 10.3, 4.3), 4.78 (2H, bs). 7.25-7.52 (5H, m)

Minor: (main peaks) 2.23 (1H, dd, J = 4.7, 2.0), 2.30 (1H, d, J = 4.4), 2.36 (3H, s), 3.16 (3H, s), 3.26 (1H, dd, J = 12.6, 2.0), 3.48 (1H, d, J = 12.8), 3.59 (1H, dd, J = 10.8, 4.7), 4.54 (1H, t, J = 10.8), 4.77 (1H, dd, J = 10.6, 4.3)

Compound 9 (80 mg) was dissolved in a mixture of chloroform (4.0 ml) and pyridine (0.5 ml). To the mixture was added 50 mg of metachloroperbenzoic acid having a purity of 70%. The material was treated in a similar manner to that described in Example 2 to give a reaction solution containing Compound 10 viz. a compound of the formula (I-3) wherein $R_1$ = $CH_2OCONH_2$, $R_2$ = H, Y = Z = $CH_3$ and n = 2] [Process-B].

At a temperature of 0°C, to this reaction solution were added sodium borohydride labelled with $^2$H (6.0 mg) and methanol-$d_4$ (0.2 ml). The reaction solution was treated in a similar manner to that described in Example 2 to obtain Compound 11 viz. a compound of the formula (I-4) wherein W = $^2$H, $R_1$ = $CH_2OCONH_2$, $R_2$ = H, Y = Z = $CH_3$ and n = 2. The formation of Compound 11 was confirmed with reference to TLC showing the same values identical to those of Compound II-2 [Process C].

Compound 11 was dissolved in methanol (2.0 ml). To the solution was then added 6.8M-ammonia in methanol solution (2.0 ml). The material was treated in a similar manner to that described in Example 2 to obtain 18.6 mg of [$C_6$-$CH_3$, $^2H_1$]porfiromycin (Compound 8) with a yield of 36% calculated on the basis of Compound 9 [Process-D].

$^1$H-NMR data revealed that the ratio of deuterium was about 80%.

EI-MS : m/z 349 ($M^+$) $C_{16}H_{19}{}^2HN_4O_5$ = 349

$^1$H-NMR: δ, ppm

1.74 (about 1.6H, t, J = 2.2), 1.76 (about 0.6H, s), 2.25 (1H, dd, J = 4.7, 2.2), 2.26 (3H, s), 2.29 (1H, d, J = 4.7), 3.18 (3H, s), 3.46 (1H, dd, J = 13.0, 2.2), 3.58 (1H, dd, J = 10.8, 4,4), 4.22 (1H, d, J = 13.0), 4.35 (1H, t, J = 10.6), 4.70 (1H, dd, J = 10.6, 4.4), 4.84 (2H, bs), 5.28 (2H, bs).

## Claims

1. Mitomycin derivatives of the following formula (I)

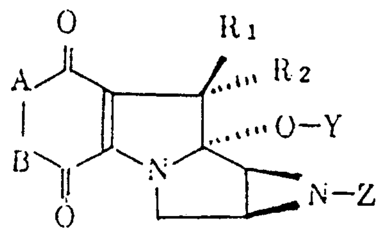

(I)

(wherein

-A-B- is 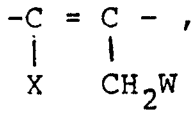 ,

or

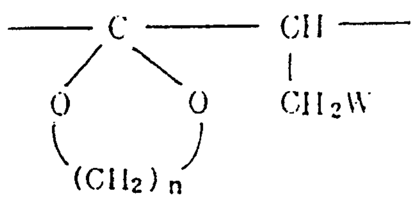

[wherein W represents deuterium ($^2H$) or tritium ($^3H$);
X represents a methoxy or amino group,
one of $R_1$ and $R_2$ represents a carbamoyloxymethyl group and the other represents a hydrogen atom; or optionally $R_1$ and $R_2$ are combined together and represent a methylene group ($=CH_2$);
Y represents a hydrogen atom or a methyl group; and
Z represents hydrogen, a methyl group or an acyl group].

2) [$C_6$ - $CH_3$, $^2H_1$]mitomycin C.

3) [$C_6$ - $CH_3$, $^3H_1$]mitomycin C.

4) [$C_6$ - $CH_3$, $^2H_1$]mitomycin A.

5) [$C_6$ - $CH_3$, $^2H_1$]porfiromycin.

6) A pharmaceutical composition comprising an isotopically labelled mitomycin derivative of any of claims 1-5 and a physiologically acceptable carrier.

7) The use of an isotopically labelled mitomycin derivative of any of claims 1-5 to trace the metabolic fate of said mitomycin in a mammal of mammalian cell.

8) A process for the preparation of compounds of the formula (I-1a) :-

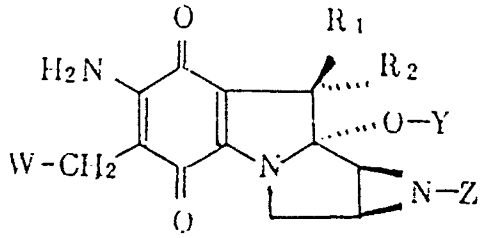

(I-1a)

[wherein W represents deuterium ($^2H$) or tritium ($^3H$); Y represents hydrogen or a methyl group; Z represents hydrogen, a methyl group or an acyl group; and one of $R_1$ and $R_2$ represents a carbamoyloxymethyl group and the other represents a hydrogen atom; or optionally $R_1$ and $R_2$ are combined together and represent a methylene group ($=CH_2$)];
which comprises reacting a compound of the formula (I-4):-

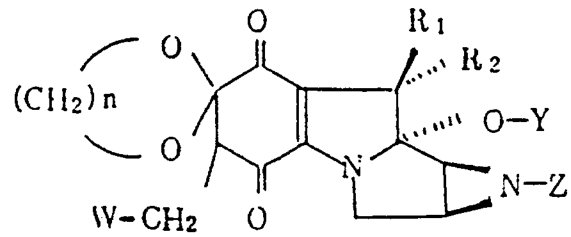

(I-4)

(wherein W, Y, Z, $R_1$ and $R_2$ are as defined above and n is an integer of 2 or 3) with ammonia.

9) A process for the preparation of compounds of the formula (I-1b):-

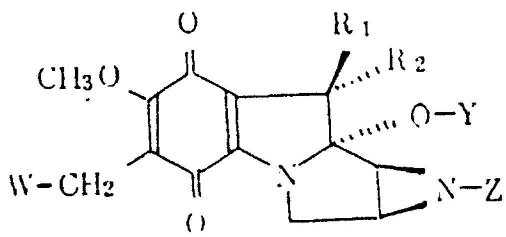

(I-1b)

[wherein W represents deuterium ($^2H$) or tritium ($^3H$);
Y represents hydrogen or a methyl group;
Z represents hydrogen, a methyl group or an acyl group; and one of $R_1$ and $R_2$ represents a carbamoyloxymethyl group and the other represents a hydrogen atom or optionally $R_1$ and $R_2$ are combined together and represent a methylene group ($=CH_2$)] which comprises reacting a compound of the formula (I-4) as defined in claim 8 with methanol in the presence of a base.

10) A process for the preparation of compounds of the formula (I-4) as defined in claim 8 which comprises reacting a compound of the formula (I-3):-

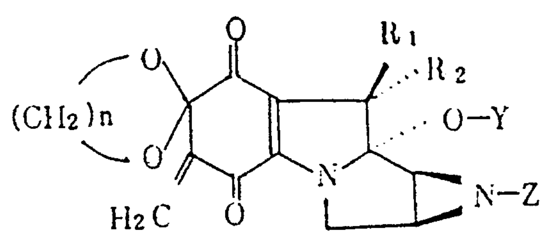

(I-3)

(wherein Y, Z, $R_1$, $R_2$ and n are as defined for I-4) with a complex hydride, e.g. sodium borohydride, labelled with deuterium ($^2H$) or tritium ($^3H$).

11) A process for the preparation of compounds of the formula (I-3) as defined in claim 10 which comprises oxidizing a compound of the formula (I-2a) :-

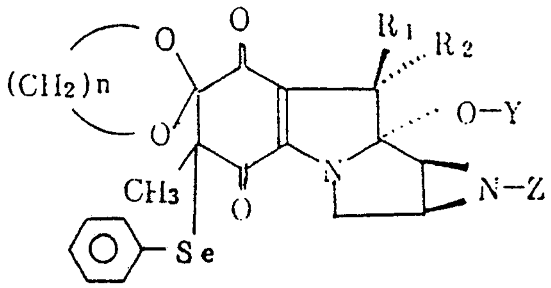

(I-2a)

(wherein Y, Z, $R_1$, $R_2$ and n are as defined for I- in the presence of an oxiding agent.

12) A process for the preparation of compounds of the formula (I-2a) as defined in claim 11 which comprises reacting a compound of the formula (II) :-

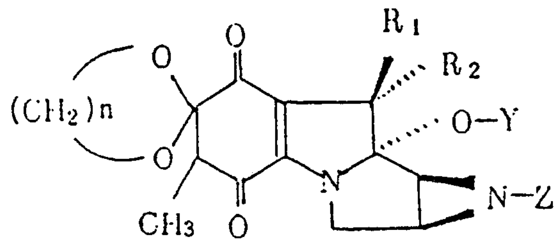

(II)

(wherein Y, Z, $R_1$, $R_2$ and n are as defined for I-2a) with a compound of the formula (III) :-

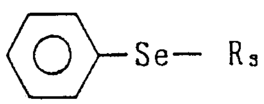

(III)

(wherein $R_3$ represents a halogen atom or an organic imide residue).

13) Mitomycin derivatives of the formula (I) as defined in claim 1 wherein -A-B- is

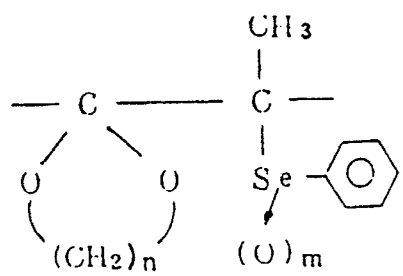

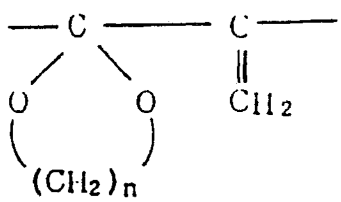

wherein n is 2 or 3, m is zero or 1, and X, Y, Z, $R_1$ and $R_2$ are as defined in claim 1.